# EUROPEAN PATENT APPLICATION

(11) **EP 4 534 555 A1**
(43) Date of publication of application: **09.04.2025**
(21) Application number: 23814688.0
(22) Date of filing: 07.03.2023
(51) Int. Cl.: C07K 14/78, C12N 15/12, C12N 15/70, C12N 1/21

(54) **PREPARATION METHOD FOR BIOSYNTHETIC HUMAN BODY STRUCTURAL MATERIAL**

(30) Priority: 01.06.2022 CN 202210619454
(71) Applicant: Shanxi Jinbo Bio-Pharmaceutical Co., Ltd., Shanxi 030032 (CN)
(72) Inventor: JIN, Xuekun, Taiyuan, Shanxi 030032 (CN); LAN, Xiaobin, Taiyuan, Shanxi 030032 (CN); WANG, Lingling, Taiyuan, Shanxi 030032 (CN); ZHANG, Yongjian, Taiyuan, Shanxi 030032 (CN); YANG, Xia, Taiyuan, Shanxi 030032 (CN); HE, Zhenrui, Taiyuan, Shanxi 030032 (CN)
(74) Representative: Aera A/S
(86) International application number: PCT/CN2023/079951
(87) International publication number: WO 2023/231489

(57) **Abstract**

Disclosed in the present invention is a preparation method for a biosynthetic human body structural material. The present invention provides a recombinant humanized collagen type IV, the amino acid sequence thereof comprising n repetitive sequences, the n being an integer greater than or equal to 1, and the repetitive sequences being functional region sequences of native human collagen type IV The present invention provides the amino acid sequence of the recombinant humanized collagen type IV capable of being correctly expressed in vitro and biosynthesis thereof is successfully performed in vitro. The amino acid sequence of said protein is derived from the native human collagen type IV and does not cause adverse immune reactions when the protein is applied to a human body; in addition, compared with commercial humanized collagen, the recombinant humanized collagen type IV prepared by the present invention has a higher cell adhesion effect. Furthermore, the preparation method provided by the present invention is simple and can produce the recombinant humanized collagen type IV with a relatively high yield at a low cost.

## Description

### PRIORITY RIGHT AND RELATED APPLICATION

The present application claims the priority to and the benefit of Chinese Patent Application No. 202210619454.1 filed with the Patent Office of CNIPA on June 1, 2022 and entitled "PREPARATION METHOD FOR BIOSYNTHETIC HUMAN BODY STRUCTURAL MATERIAL", the entire contents of which are incorporated herein by reference.

### TECHNICAL FIELD

The present disclosure pertains to the field of biotechnology, and specifically relates to a preparation method for a biosynthetic human body structural material.

### BACKGROUND

Structural materials of the human body are primarily the structural protein including collagen. This kind of protein provides the adhesive and supportive functions to cells and tissue, and is the main component of the extracellular matrix.

Collagen is a protein widely distributed in the connective tissue of the human body, and is also the most abundant protein in the human body, accounting for 25% to 35% of the total proteins. At least 28 subtypes of collagen have currently been found in the human body, which are located in different tissue and organs. Among them, type IV collagen is present mainly in the vascular areas in the hepatic portal vein, around the central veins, and distributed along the sinusoid, and is a major constituent of the scaffold of the basement membranes. It has a nonfibrillar reticular collagen triple-helical structure, functions as an extracellular matrix, and is able to support cells. Collagen is a native biological resource with the biological histocompatibility that other polymers cannot match. Therefore, collagen can be used as an important structural material of the human body and widely used in high-end pharmaceutical industry.

However, collagen raw materials are mainly extracted from animal tissue at present. Unfortunately, the content of type IV collagen in animals is low, which makes it impossible to extract single-component type IV collagen. At the same time, the animal-derived immune response is also a major cause for the restricted applications of collagen. With the growing collagen industry in China, the techniques of preparing collagen by biosynthetic pathways are becoming more and more mature, and especially humanized collagen has been at the forefront of the world. In 2021, the National Medical Products Administration named and classified the biosynthetic collagen. Among them, the recombinant humanized collagen refers to a full-length or partial amino acid sequence fragment encoded by a gene of a specific type of human collagen prepared by DNA recombinant technology, or a combination of functional fragments containing human collagen.

In organisms, type IV collagen has a nonfibrillar triple-helical structure, and has six genetically different α chains: α1(IV) to α6(IV), which form three different molecular forms in tissue, *i.e.,* α1α1α2(IV), α3α4α5(IV), and α5α5α6(IV) that could be selectively expressed in different membranes in particular tissue at different stages. Structurally, the structure of native type IV collagen of the human body is very complicated, which is the reason that the humanized collagen is extremely difficult to express and mass-produce by conventional means. Synthesis and modification of collagen begins with procollagen, undergoes many chemical changes such as hydroxylation, glycosylation, and crosslinking, and is subject to intricate regulations by a variety of biological enzymes. In addition to the collagen chain, procollagen further contains spherical heads and tails. Without these heads and tails, the collagen chain would not fold into a correct triple helix and would thus lack the biological activity of collagen. Therefore, collagen prepared according to the original gene sequence cannot be spontaneously organized to form a correct spatial structure *in vitro.* Such difficulties seriously hinder the development and production of humanized collagen.

Conventional methods for producing collagen are to treat animal-derived tissue by acidolysis, alkaline hydrolysis, and enzymolysis and extract collagen derivatives. The collagen itself extracted by these methods has lost its original biological activity and thus cannot be applied to the biomedical field to fulfill its true function. With the development of modern biotechnology, people are constantly trying to utilize transgenic techniques to prepare recombinant human collagen in animal, plant, and microbial expression systems, which solves many shortcomings of the conventional extraction processes. However, there are also research institutes indicating that the receptors expressible in human B cells are not expressed in mice, so this suggests that humanized models can provide expression unavailable in conventional mice. Therefore, there is an urgent need for a biosynthetic method for humanized type IV collagen that can overcome this shortcoming, so that it can be widely used as a structural material of the human body.

### SUMMARY

### Technical Problem

The present disclosure is intended to provide a recombinant type IV humanized collagen, and provide a biosynthetic preparation method therefor, and use thereof in the field of medicine to resolve the problems that native human type IV collagen cannot be correctly expressed and mass-produced *in vitro,* so as to provide new possibilities for production and applications of humanized collagen.

### Solution to Problem

According to the first aspect of the present disclosure, there is provided a recombinant type IV humanized collagen. Specifically, the present disclosure screens out a sequence of a functional domain of native human type IV collagen, and uses it as a repeating sequence, so that the amino acid sequence of the recombinant type IV humanized collagen comprises n repeating sequence, *i.e.,* the sequence of the functional domain of the native human type IV collagen, wherein n is an integer equal to or greater than 1. The recombinant type IV humanized collagen is composed of a sequence of the functional domain of the native human type IV collagen.

Further, n may be 1, 2, 3, 4, 5, 6, 7 or 8, wherein when n is an integer equal to or greater than 2, each of the repeating sequences is directly linked, so as to obtain the recombinant type IV humanized collagen having an amino acid sequence comprising a sequence set forth in SEQ ID NO.1, SEQ ID NO.2, or SEQ ID NO.3.

Furthermore, for ease of expression and purification of the recombinant type IV humanized collagen in practical production process, the amino acid sequence of the recombinant type IV humanized collagen provided in the present disclosure may further comprise an amino acid sequence excisable by a TEV protease, and the amino acid sequence excisable by the TEV protease is set forth in SEQ ID NO.7; optionally, the amino acid sequence excisable by the TEV protease is directly linked to the repeating sequence; preferably, the amino acid sequence excisable by the TEV protease is at the N-terminus of the recombinant type IV humanized collagen.

The present disclosure further provided a recombinant type IV humanized collagen comprising the above-mentioned amino acid sequence as a core domain.

According to the second aspect of the present disclosure, there is provided a polynucleotide encoding the recombinant type IV humanized collagen according to the first aspect of the present disclosure.

Furthermore, the sequence of the polynucleotide may comprise a sequence set forth in SEQ ID NO.4, SEQ ID NO.5, or SEQ ID NO.6, or a derivative thereof after artificial design and codon optimization.

According to the third aspect of the present disclosure, there is provided a series of recombinant expression vectors comprising the polynucleotide according to the second aspect of the present disclosure. The expression vectors selected in the present disclosure can be stably present and autonomously replicable in various hosts of prokaryotic or eukaryotic cells, for example, conventional plasmids (pET series), shuttle vectors PNV 18.1, phages, or viral vectors in this field. The nucleotide sequence according to the second aspect of the present disclosure is cloned into a vector by a molecular biological manipulation such as enzyme digestion and ligation on the vector to construct a recombinant expression vector.

According to the fourth aspect of the present disclosure, there is provided a series of recombinant host cells comprising the recombinant expression vector according to the third aspect of the present disclosure. The host cells are selected from prokaryotic cells, yeast or eukaryotic cells, and may be further selected from *E. coli, Rhodococcus ruber, Bacillus subtilis,* and *Saccharomyces.* The recombinant expression vector according to the third aspect of the present disclosure is transformed into a host cell to obtain a corresponding genetically engineered bacterium.

According to the fifth aspect of the present disclosure, there is provided a preparation method for the recombinant type IV humanized collagen according to the first aspect of the present disclosure, which may comprise the following steps:
S1: subjecting the recombinant host cell according to the fourth aspect of the present disclosure to fermentation culture, and inducing expression of a protein; in this step, the fermentation culture may be carried out in a shaker at a rotational speed of 200 to 240 rpm and at a temperature of 35°C to 38°C, preferably at a rotational speed of 220 rpm, preferably at a temperature of 37°C; the specific procedure of inducing the expression of the protein may be as follows: cooling to 16°C to 30°C and adding IPTG; optionally, the IPTG has a working concentration of 0.3 to 0.7 mM, preferably 0.5 mM.
S2: collecting the protein; in this step, the specific procedure of collecting the protein may be as follows: centrifuging the mixture obtained in step S1 at 5000 to 7000 rpm for 10 to 15 min at 4°C, and re-suspending the precipitate with an aqueous solution containing 20 to 30 mM Tris, 150 to 250 mM sodium chloride, and 15 to 25 mM imidazole, and centrifuging at 15000 to 18000 rpm for 20 to 40 min at 4°C after high-pressure homogenization or ultrasonic disruption of the cells; preferably, the specific procedure of collecting the protein may be as follows: centrifuging the mixture obtained in step S1 at 6000 rpm for 12 min at 4°C, re-suspending the precipitate with an aqueous solution containing 25 mM Tris, 200 mM sodium chloride, and 20 mM imidazole, and cooling to ≤15°C, and centrifuging at 17000 rpm for 30 min at 4°C after high-pressure homogenization or ultrasonic disruption of the cells; and
S3: purifying the protein; preferably, this step comprises purifying the protein by a Ni affinity chromatography column and/or an ion exchange chromatography column, and optionally subjecting the protein to enzyme digestion; preferably, the protein is enzyme digested by a TEV protease; more preferably, the mass ratio of the protein to the TEV protease is 15:1 to 25:1.

Furthermore, prior to the step S1, the preparation method may further comprise a step of screening a functional domain of the native human type IV collagen, a step of constructing a recombinant expression vector, and a step of constructing a recombinant host cell. Specifically, the specific procedure of the step of screening a functional domain of the native human type IV collagen may be as follows: excluding an uncharged amino acid motif from a helical domain of the native human type IV collagen, subsequently screening out a potential helical functional domain with the most interchain hydrogen bonds and the best ability to stabilize a trimeric aggregation form by the computer-assisted protein structure prediction method, and thereafter screening out the functional domain of the native human type IV collagen with the highest protein expression level, ease of purification, and good stability based on the prediction method for protein expression properties. By repeatedly linking the amino acid sequence of the screened functional domain of the native human type IV collagen and optionally adding an amino acid sequence excisable by the TEV protease, the amino acid sequence of the recombinant type IV humanized collagen according to the first aspect of the present disclosure is obtained. Subsequently, the polynucleotide sequence of the coding gene of the above recombinant type IV humanized collagen may be artificially designed and optimized according to the codon preference of the target organism to obtain the nucleotide sequence according to the second aspect of the present disclosure, which is cloned into a vector to construct the recombinant expression vector according to the third aspect of the present disclosure. The above vector is transformed into a host cell to obtain the recombinant host cell according to the fourth aspect of the present disclosure.

According to the sixth aspect of the present disclosure, there is provided use of the recombinant type IV humanized collagen according to the first aspect of the present disclosure in the preparation of a product including a medical device, a cosmetic, a healthcare product, or a medicament.

Specifically, the present disclosure is described as follows:
[1]. A recombinant type IV humanized collagen, wherein an amino acid sequence of the recombinant type IV humanized collagen comprises n repeating sequence, n being an integer equal to or greater than 1; wherein the repeating sequence is a sequence of a functional domain of a native human type IV collagen;
   preferably, n is 1, 2, 3, 4, 5, 6, 7 or 8, wherein when n is an integer equal to or greater than 2, each of the repeating sequences is directly linked;
   more preferably, the amino acid sequence of the recombinant type IV humanized collagen comprises any one of the following (i) to (iii):
      (i) an amino acid sequence set forth in SEQ ID NO.1, SEQ ID NO.2, or SEQ ID NO.3;
      (ii) an amino acid sequence obtained by adding, substituting, deleting or modifying one or more amino acid residues in the amino acid sequence set forth in SEQ ID NO.1, SEQ ID NO.2, or SEQ ID NO.3 and retaining a cell adherence effect of the native human type IV collagen; and
      (iii) an amino acid sequence that is encoded by a nucleotide sequence hybridized under a stringency condition with a polynucleotide sequence encoding the sequence set forth in SEQ ID NO.1, SEQ ID NO.2, or SEQ ID NO.3, and retains the cell adherence effect of the native human type IV collagen, the stringency condition being a medium stringency condition, a medium-high stringency condition, a high stringency condition or a very high stringency condition.
[2]. The recombinant type IV humanized collagen according to [1], wherein the amino acid sequence of the recombinant type IV humanized collagen further comprises an amino acid sequence excisable by a TEV protease, and the amino acid sequence excisable by the TEV protease is set forth in SEQ ID NO.7;
   preferably, the amino acid sequence excisable by the TEV protease is directly linked to the repeating sequence;
   preferably, the amino acid sequence excisable by the TEV protease is at N-terminus of the recombinant type IV humanized collagen.
[3]. A polynucleotide encoding the recombinant type IV humanized collagen according to [2],
   preferably, a sequence of the polynucleotide comprising a sequence set forth in SEQ ID NO.4, SEQ ID NO.5, or SEQ ID NO.6.
[4]. A recombinant expression vector, wherein the recombinant expression vector comprises the polynucleotide according to [3],
   preferably, the recombinant expression vector comprises a pET series vector, a shuttle vector, a phage or a viral vector;
   more preferably, the recombinant expression vector is pET-28a-Trx-His.
[5]. A recombinant host cell, wherein the recombinant host cell comprises the recombinant expression vector according to [4],
   preferably, the recombinant host cell is a prokaryotic cell, yeast or a eukaryotic cell;
   more preferably, the recombinant host cell is *E. coli* BL21(DE3).
[6]. A preparation method for the recombinant type IV humanized collagen according to [1] or [2] comprising the following steps:
   S1: subjecting the recombinant host cell according to [5] to fermentation culture, and inducing expression of a protein;
   S2: collecting the protein; and
   S3: purifying the protein; preferably, comprising purifying the protein by a Ni affinity chromatography column and/or an ion exchange chromatography column, and optionally subjecting the protein to enzyme digestion, preferably subjecting the protein to enzyme digestion by a TEV protease, more preferably, a mass ratio of the protein to the TEV protease being 15:1 to 25:1.
[7]. The preparation method according to [6], wherein in the step S1, the fermentation culture is carried out in a shaker at a rotational speed of 200 to 240 rpm and at a temperature of 35°C to 38°C.
[8]. The preparation method according to [6] or [7], wherein in the step S1, a specific procedure of inducing the expression of the protein is as follows: cooling to 16°C to 30°C and adding IPTG; preferably, the IPTG has a working concentration of 0.3 to 0.7 mM.
[9]. The preparation method according to any one of [6] to [8], wherein in the step S2, a specific procedure of collecting the protein is as follows: centrifuging a mixture obtained in the step S1 at 5000 to 7000 rpm for 10 to 15 min at 4°C, and re-suspending a precipitate with an aqueous solution containing 20 to 30 mM Tris, 150 to 250 mM sodium chloride, and 15 to 25 mM imidazole, and centrifuging at 15000 to 18000 rpm for 20 to 40 min at 4°C after high-pressure homogenization or ultrasonic disruption of the cells.
[10]. Use of the recombinant type IV humanized collagen according to [1] or [2] in a preparation of a product including a medical device, a cosmetic, a healthcare product, or a medicament.

### Advantageous Effect of the Invention

By implementing the above technical solutions, the present disclosure provides the amino acid sequence of the recombinant type IV humanized collagen capable of being correctly expressed *in vitro,* and has successfully biosynthesized it *in vitro.* The amino acid sequence of this protein is derived from native human type IV collagen, and does not cause any adverse immune response when used in the human body. Furthermore, compared with the commercial humanized collagen, the recombinant type IV humanized collagen prepared in the present disclosure has a higher cell adherence effect. Meanwhile, the preparation method provided in the present disclosure is simple and allows for a higher yield of the recombinant type IV humanized collagen at a low cost.

### BRIEF DESCRIPTION OF THE DRAWINGS

FIG. 1 shows the electrophoresis test results involved in the expression and purification processes of the recombinant type IV humanized collagen C007.
FIG. 2 shows the electrophoresis test results involved in the expression and purification processes of the recombinant type IV humanized collagen C4P7C6.
FIG. 3 shows the electrophoresis test results involved in the expression and purification processes of the recombinant type IV humanized collagen C4P7E5.
FIG. 4 shows the comparison results of stability of the expression and purification between the recombinant type IV humanized collagen C4P7C6 and the recombinant type IV humanized collagen C007.
FIG. 5 shows the comparison results of the cell adherence activities of different recombinant type IV humanized collagens.
FIG. 6 shows the vector spectrum of the expression vector pET-28a-Trx-His.

### DETAILED DESCRIPTION

The embodiments of the present disclosure will be illustrated below, but the present disclosure is not limited thereto.

The term "may" used in the present disclosure involves both the meaning of doing something and the meaning of not doing something.

In the present disclosure, the term "optional" or "optionally" means that the event or case described subsequently may or may not occur, and the description includes the case where the event occurs and the case where the event does not occur.

In the present disclosure, the term "including", "having", "comprising", or "containing" is intended to be inclusive or open-ended, and does not exclude additional or unrecited elements or methods and steps. At the same time, the term "including", "having", "comprising", or "containing" may also be intended to be close-ended and exclude additional or unrecited elements or methods and steps.

The numerical ranges represented by "numerical value A to numerical value B", "numerical value A or more", and "numerical value A or less" used in the present disclosure refers to the range including the endpoint values A and B or the range including the endpoint value A.

In the present disclosure, an arbitrary numerical value includes the standard deviation of the error caused by the device or method used for determining the value. All the numerical ranges and parameters used to define the present disclosure are approximate numerical values, and the relevant numerical values in the specific examples have been presented herein as precisely as possible. However, any numerical value inherently and inevitably contains a standard deviation caused by the aforementioned test device or method.

In the present disclosure, the terms "polypeptide" and "protein" may interchangeably refer to a string of at least two amino acid residues linked to each other via a covalent bond (such as a peptide bond), and may be a recombinant polypeptide, a native polypeptide or a synthetic polypeptide. The polypeptide may be linear or branched. It may comprise modified amino acids, and may be interrupted by non-amino acids. The term also includes amino acid polymers that have been modified (*e.g*., disulfide bond formation, glycosylation, lipidation, acetylation, phosphorylation, or any other manipulations, such as conjugation with a labeling component).

In the present disclosure, the term "amino acid" may include natural amino acids, unnatural amino acids, amino acid analogs, and all D and L stereoisomers thereof.

In the present disclosure, amino acid deletion may refer to the deletion of 1, 2, or 3 or more amino acids from an amino acid sequence, as long as the altered sequence fully or partially retains the activity of the original amino acid sequence.

In the present disclosure, amino acid addition may refer to the addition of 1, 2, or 3 or more amino acids at the C-terminus, N-terminus, or any position between the C-terminus and N-terminus of an amino acid sequence, as long as the altered sequence fully or partially retains the activity of the original amino acid sequence.

In the present disclosure, amino acid substitution may refer to the substitution of an amino acid at a certain position of an amino acid sequence with an additional amino acid, as long as the altered sequence fully or partially retains the activity of the original amino acid sequence. Amino acid substitutions may be conservative amino acid substitutions, which mean that several amino acids are substituted with amino acids of similar or approximate properties to form peptides (conservative variant peptides) as compared to the original amino acid sequence. Exemplarily, these conservative variant peptides may be produced by the following substitutions of amino acids: a substitution of Ala with Val, Leu or Ile, a substitution of Arg with Lys, Gln, Asn or His, a substitution of Asn with Gln, His, Lys or Arg, a substitution of Asp with Glu or Asn, a substitution of Cys with Ser or Ala, a substitution of Gln with Asn or Glu, a substitution of Glu with Asp or Gln, a substitution of Gly with Ala, a substitution of His with Asn, Lys, Gln or Arg, a substitution of Ile with Leu, Met, Ala, Val or Phe, a substitution of Leu with Ile, Met, Ala, Val or Phe, a substitution of Lys with Asn, Gln or Arg, a substitution of Met with Ile, Leu or Phe, a substitution of Phe with Leu, Val, Ile, Ala or Tyr, a substitution of Pro with Ala, a substitution of Ser with Thr, a substitution of Thr with Ser or Val, a substitution of Trp with Phe or Tyr, a substitution of Tyr with Trp, Phe, Thr or Ser, and a substitution of Val with Phe, Ala, Met, Ile or Leu. Amino acid substitutions may also be non-conservative amino acid substitutions.

In the present disclosure, amino acid modifications may include modifications to native sequences, such as modifications to functional groups, intramolecular covalent bonding (*e.g*., ring formation between side chains), methylation, acylation, ubiquitination, phosphorylation, aminohexanylation, and biotinylation.

In the present disclosure, "hybridization" means the ability of a polynucleotide or oligonucleotide to bind to a substantially complementary sequence under stringency conditions where non-specific binding to a non-complementary object does not occur. In this regard, the sequences are preferably 90% to 100% complementary. The properties of complementary sequences capable of specifically binding to each other are applied, for example, in Northern or Southern blotting techniques, or in PCR or RT-PCR primer binding. According to the present disclosure, the hybridization occurs under medium stringency conditions, medium-high stringency conditions, high stringency conditions or very high stringency conditions. Such hybridization conditions are described in Current Protocols in Molecular Biology, John Wiley&Sons, N.Y. (1989), 6.3.1-6.3.6. For example, the specific hybridization conditions are as follows: (1) the low stringency hybridization conditions refer to 6× sodium chloride/sodium citrate (SSC) at about 45°C, and then washing twice in 0.2× SSC and 0.1% SDS at 50°C or higher (for the low stringency conditions, the washing temperature can be raised to 55°C); (2) the medium stringency hybridization conditions refer to 6× SSC at about 45°C, then washing one or more times in 0.2× SSC and 0.1% SDS at 60°C; (3) the high stringency hybridization conditions refer to 6× SSC at about 45°C, and then washing one or more times in 0.2× SSC and 0.1% SDS at 65°C, which are preferred; and (4) the very high stringency hybridization conditions refer to 0.5 M sodium phosphate and 7% SDS at 65°C, and then washing one or more times in 0.2× SSC and 1% SDS at 65°C.

In the present disclosure, suitable vectors are those known in the art of vector construction, including selection of promoters and other regulatory elements, such as enhancer elements. The vectors according to the present disclosure include the sequences suitable for introduction into cells. For example, the vector may be an expression vector, in which the coding sequence of the protein is under the control of its own cis-acting regulatory elements, and the vector is designed to facilitate gene integration or gene replacement in the host cell, etc. One of ordinary skill in the art shall be appreciated that in the present disclosure, the "vector" includes a DNA molecule, for example, a plasmid, phage, virus, or additional vector, which contains one or more heterologous or recombinant nucleotide sequences. Suitable phages and viral vectors include, but are not limited to: λ-phage, EMBL phage, Simian virus, bovine papillomavirus, Epstein-Barr virus, adenovirus, herpes virus, mouse sarcoma virus, murine breast cancer virus, lentivirus, and the like.

In the present disclosure, the host cell may be a eukaryotic cell, *e.g.,* fungi and yeast, a prokaryotic cell, *e.g., Enterobacteriaceae* bacteria.

In the present disclosure, the "functional domain of native human type IV collagen" refers to a sequence fragment in the native human type IV collagen α1 that has the functions (*e.g*., cell adherence activity) of the amino acid sequence of collagen.

The recombinant type IV humanized collagen provided in the present disclosure is applied in the fields including preparation of high-end medical devices, such as biological dressings, human bionic materials, plastic surgery materials, organoid culture, cardiovascular scaffolds, coatings, tissue injection filling, ophthalmic materials, obstetrics and gynecology biomaterials, nerve repair and regeneration, liver tissue and blood vessel repair and regeneration, and 3D-printed artificial organ biomaterials; and high-end cosmetic raw materials, high-end healthcare products, high-end pharmaceutical excipients, etc.

### Examples

The present disclosure is further illustrated with reference to the following examples, but any examples or combinations thereof shall not be construed as limiting the scope or implementations of the present disclosure. The scope of the present disclosure is defined by the appended claims, and the scope defined by the claims will be apparent to one of ordinary skill in the art by combining the present specification and the general knowledge in the art. Without departing from the spirit and scope of the present disclosure, a person skilled in the art can make any modifications or changes to the technical solutions of the present disclosure, and such modifications and changes are also encompassed in the scope of the present disclosure.

Where specific conditions are not indicated in the examples, conventional conditions or the conditions recommended by the manufacturers shall be followed. Where the manufacturers of the reagents or instruments used are not indicated, all of them are commercially-available conventional products. For a better illustration of the present disclosure, numerous details are provided in the specific embodiments below. It should be appreciated to a person skilled in the art that the present disclosure can also be implemented even without those details. In other examples, the methods, means, devices, and steps well known to a person skilled in the art are not descried in detail, so as to make the principle of the present disclosure apparent.

Unless otherwise defined, all technical and scientific terms used herein have the same meanings as typically understood by one of ordinary skill in the art. Unless otherwise specified, all the units used in the present specification are international standard units, and all the numerical values and numerical ranges used in the present disclosure shall be construed as including inevitable systematic errors.

### Example 1: Construction and Expression of Recombinant Type IV Humanized Collagens

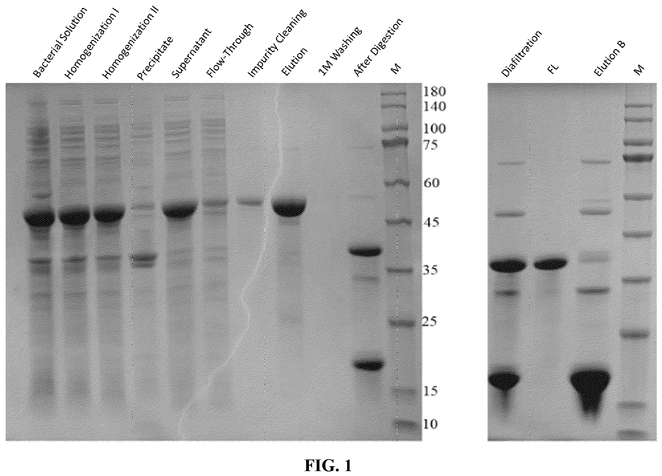

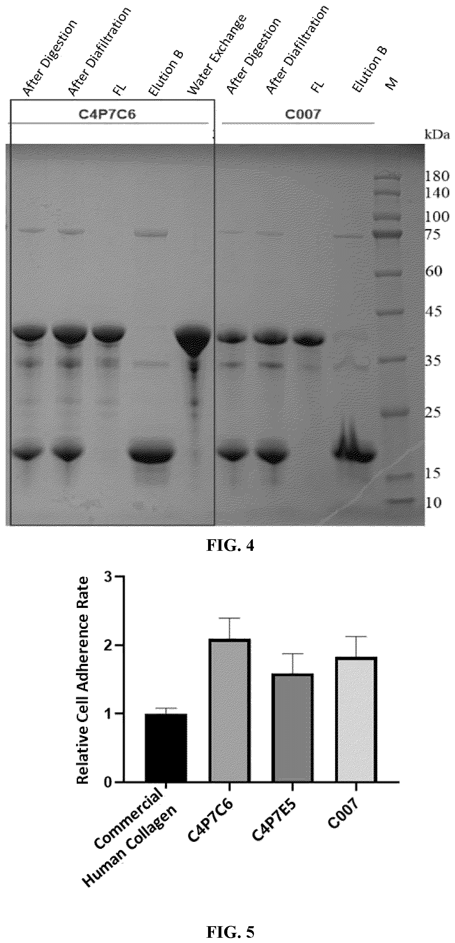

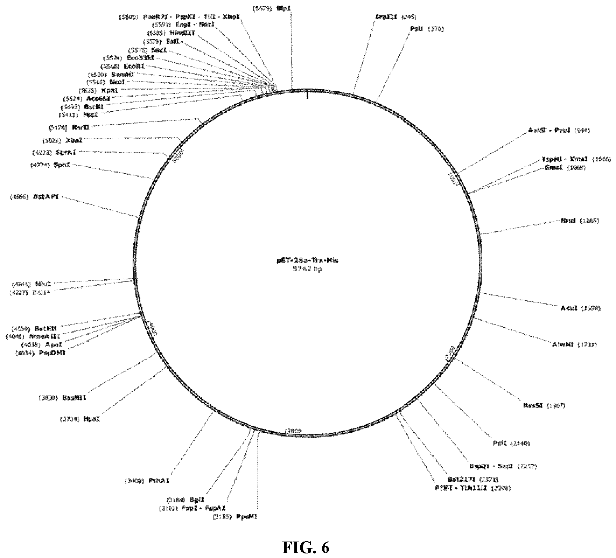

1. Large-scale screening of a functional domain of the protein was carried out: 1) sequence screening: the Gly-X-Y repeating sequence in the helical domain of the native type IV collagen contained a large number of charged amino acids, and the charges bound to cells through interaction, so the domains that did not contain these important charge motifs were excluded; 2) a potential helical functional domain with the most interchain hydrogen bonds and the best ability to stabilize a trimeric aggregation form was screened out with the aid of the computer-assisted protein structure prediction method; 3) the functional domain of the human type IV collagen with the highest protein expression level, ease of purification, and good stability was screened out based on the prediction method for protein expression properties; and 4) the amino acid fragments of the screened domain were optimized by n repetitions of direct linkage in order to ensure that the recombinant type IV humanized collagen had a molecular weight within a certain range and was easily purified and stabilized; finally, the following target fragments of different recombinant type IV humanized collagens were obtained:
   (1) the amino acid sequence of the recombinant type IV humanized collagen C007: 
GQKGDQGEKGQIGPIGEKGSRGDPGTPGVPGKDGQAGQPGQPGPKGDPGISGTPGAPG
LPGPKGSVGGMGLPGTPGEKGVPGIPGPQGSPGLPGDKGAKGEKGQAGPPGIGIPGLR
GEKGDQGIAGFPGSPGEKGEKGSIGIPGMPGSPGLKGSPGSVGYPGSPGLPGEKGDKGL
PGLDGIPGVKGEAGLPGTPGPTGPAGQKGEPGSDGIPGSAGEKGEPGLPGRGFPGFPGA
KGDKGSKGEVGFPGLAGSPGIPGSKGEQGFMGPPGPQGQPGLPGSPGHATE (SEQ ID
NO.1);
   (2) the amino acid sequence of the recombinant type IV humanized collagen C4P7C6: 
GFPGFPGAKGDKGSKGEVGFPGLAGSPGIPGSKGEQGFMGPPGPQGQPGLPGSPGHAG
FPGFPGAKGDKGSKGEVGFPGLAGSPGIPGSKGEQGFMGPPGPQGQPGLPGSPGHAGF
PGFPGAKGDKGSKGEVGFPGLAGSPGIPGSKGEQGFMGPPGPQGQPGLPGSPGHAGFP
GFPGAKGDKGSKGEVGFPGLAGSPGIPGSKGEQGFMGPPGPQGQPGLPGSPGHAGFPG
FPGAKGDKGSKGEVGFPGLAGSPGIPGSKGEQGFMGPPGPQGQPGLPGSPGHAGFPGF
PGAKGDKGSKGEVGFPGLAGSPGIPGSKGEQGFMGPPGPQGQPGLPGSPGHA (SEQ ID
NO.2); and
   (3) the amino acid sequence of the recombinant type IV humanized collagen C4P7E5: 
GLPGTPGPTGPAGQKGEPGSDGIPGSAGEKGEPGLPGRGFPGFPGAKGDKGSKGEVGF
PGLAGSPGIPGSKGEQGFMGLPGTPGPTGPAGQKGEPGSDGIPGSAGEKGEPGLPGRGF
PGFPGAKGDKGSKGEVGFPGLAGSPGIPGSKGEQGFMGLPGTPGPTGPAGQKGEPGSD
GIPGSAGEKGEPGLPGRGFPGFPGAKGDKGSKGEVGFPGLAGSPGIPGSKGEQGFMGL
PGTPGPTGPAGQKGEPGSDGIPGSAGEKGEPGLPGRGFPGFPGAKGDKGSKGEVGFPG
LAGSPGIPGSKGEQGFM (SEQ ID NO.3).
   When the recombinant type IV humanized collagens of the present disclosure are expressed, the amino acid sequence ENLYFQ (SEQ ID NO.7) excisable by the TEV protease could be added at the N-terminus thereof. Preferably, the amino acid sequence excisable by the TEV protease was directly linked to the sequence of the functional domain of the native human type IV collagen.
2. The synthesized polynucleotide fragments were inserted into the pET-28a-Trx-His expression vector (with the vector spectrum shown in FIG. 6) respectively to obtain the corresponding recombinant expression plasmids. The polynucleotide fragments of the recombinant type IV humanized collagens were as shown below:
   (1) the polynucleotide sequence of the recombinant type IV humanized collagen C007 added with an amino acid sequence excisable by the TEV protease at the N-terminus thereof: 
GAAAACCTGTATTTCCAGGGTCAGAAAGGAGATCAAGGAGAAAAAGGACAGATAG
GACCAATAGGAGAAAAAGGGAGCCGGGGAGATCCGGGAACACCGGGAGTGCCGG
GAAAAGATGGACAGGCAGGCCAGCCGGGACAGCCGGGTCCTAAAGGAGATCCGGG
TATAAGCGGAACACCGGGTGCACCGGGATTACCTGGTCCGAAAGGGAGCGTAGGG
GGAATGGGACTGCCGGGAACACCTGGTGAAAAAGGCGTACCTGGTATTCCGGGTCC
TCAGGGGTCCCCGGGACTGCCTGGTGATAAAGGTGCAAAAGGGGAAAAAGGGCAA
GCAGGTCCGCCGGGTATTGGTATACCGGGTCTGCGTGGGGAAAAAGGTGATCAGGG
TATTGCCGGTTTTCCGGGTAGCCCGGGTGAGAAAGGTGAAAAAGGAAGTATAGGAA
TACCGGGTATGCCTGGTAGTCCGGGTCTGAAAGGTAGCCCGGGAAGCGTGGGGTAT
CCGGGTAGCCCTGGTCTGCCTGGGGAAAAAGGCGATAAAGGTCTGCCGGGTCTGG
ACGGTATTCCTGGTGTTAAAGGGGAAGCAGGTCTGCCGGGGACCCCGGGTCCAAC
AGGTCCTGCAGGGCAGAAAGGTGAACCAGGTAGCGATGGTATTCCGGGGTCTGCG
GGGGAAAAAGGAGAACCGGGTCTGCCGGGAAGAGGTTTTCCGGGCTTTCCGGGTG
CAAAAGGAGATAAGGGTAGCAAAGGCGAAGTGGGTTTTCCGGGACTGGCAGGTAG
TCCGGGAATCCCTGGTAGCAAAGGTGAACAGGGTTTTATGGGGCCGCCGGGTCCGC
AGGGTCAGCCTGGTTTACCGGGTAGCCCAGGTCATGCGACAGAG (SEQ ID NO.4);
   (2) the polynucleotide sequence of the recombinant type IV humanized collagen C4P7C6 added with an amino acid sequence excisable by the TEV protease at the N-terminus thereof: 
GAAAATCTATATTTCCAAGGATTTCCCGGGTTCCCGGGTGCAAAAGGCGACAAGGG
TTCGAAAGGCGAAGTTGGCTTCCCGGGATTAGCGGGTAGCCCAGGTATCCCGGGTT
CTAAGGGTGAACAGGGTTTCATGGGTCCGCCCGGCCCACAGGGTCAACCGGGCCT
GCCTGGTAGCCCGGGTCATGCGGGCTTTCCAGGTTTCCCGGGCGCGAAAGGCGACA
AAGGTAGCAAGGGCGAAGTCGGTTTTCCGGGCCTTGCTGGCAGCCCGGGCATCCCG
GGGTCCAAGGGCGAGCAAGGATTCATGGGTCCGCCTGGCCCACAAGGCCAACCGG
GTTTGCCGGGCTCTCCGGGTCACGCCGGTTTTCCGGGATTCCCGGGAGCGAAAGGT
GATAAGGGCTCCAAAGGTGAGGTGGGTTTTCCGGGCTTGGCTGGCAGCCCGGGTAT
TCCGGGTAGCAAGGGCGAGCAGGGTTTTATGGGCCCGCCTGGTCCGCAGGGCCAA
CCGGGGCTGCCGGGCTCCCCGGGCCACGCAGGCTTCCCAGGTTTTCCGGGTGCGA
AAGGCGATAAAGGTAGTAAGGGTGAAGTGGGCTTCCCGGGTCTCGCGGGTAGCCC
GGGTATTCCGGGCTCGAAGGGCGAACAGGGCTTTATGGGCCCACCGGGTCCGCAGG
GTCAGCCGGGCCTGCCGGGTAGCCCGGGCCACGCGGGTTTCCCGGGCTTCCCGGGT
GCCAAAGGCGATAAAGGTTCAAAGGGGGAGGTGGGTTTTCCGGGTCTGGCAGGCA
GCCCGGGCATCCCGGGCAGCAAGGGCGAACAAGGTTTTATGGGTCCGCCTGGACC
GCAAGGTCAGCCGGGCCTGCCGGGTAGTCCGGGCCATGCCGGCTTCCCGGGTTTTC
CGGGTGCGAAAGGGGACAAAGGCAGCAAGGGAGAGGTTGGTTTCCCAGGCCTGG
CTGGTAGCCCGGGTATTCCGGGCTCCAAGGGTGAGCAGGGTTTCATGGGTCCGCCT
GGCCCACAGGGTCAACCTGGCCTGCCGGGTTCTCCGGGTCATGCG (SEQ ID NO.5);
 and
   (3) the polynucleotide sequence of the recombinant type IV humanized collagen C4P7E5 added with an amino acid sequence excisable by the TEV protease at the N-terminus thereof: 
GAAAATTTATATTTTCAAGGGCTACCCGGAACGCCAGGTCCAACTGGTCCGGCGGG
CCAAAAAGGCGAGCCGGGGTCGGACGGCATCCCGGGTAGCGCAGGCGAAAAAGG
TGAACCGGGCCTGCCGGGCCGTGGCTTCCCGGGCTTCCCGGGAGCGAAAGGCGAT
AAAGGCAGCAAGGGCGAGGTGGGTTTTCCGGGATTGGCTGGTAGCCCGGGGATTC
CGGGCTCCAAGGGCGAGCAGGGTTTTATGGGTCTGCCGGGAACCCCAGGTCCGAC
CGGTCCGGCGGGTCAGAAAGGTGAACCGGGTAGCGACGGCATTCCGGGCAGCGCG
GGTGAAAAAGGTGAACCGGGTTTGCCGGGTCGCGGTTTTCCGGGTTTCCCGGGTGC
TAAAGGCGACAAAGGTTCCAAGGGTGAAGTTGGTTTTCCGGGACTGGCGGGCAGC
CCGGGCATCCCAGGCTCCAAGGGCGAACAAGGCTTTATGGGTCTGCCGGGCACCCC
GGGTCCGACCGGTCCTGCGGGTCAAAAGGGTGAGCCGGGTAGCGATGGTATTCCGG
GCTCTGCTGGCGAGAAAGGTGAGCCGGGCCTGCCGGGCCGTGGTTTCCCGGGATTC
CCTGGTGCCAAGGGTGACAAGGGTTCGAAGGGCGAGGTTGGTTTTCCGGGCCTGG
CGGGTTCTCCGGGTATCCCGGGTAGCAAGGGGGAGCAGGGTTTTATGGGCTTACCG
 
GGAACCCCGGGCCCAACGGGTCCGGCAGGTCAGAAAGGGGAACCGGGCAGCGAT
GGTATCCCGGGCAGTGCCGGTGAGAAGGGCGAGCCGGGCCTGCCCGGCCGTGGTT
TCCCTGGTTTCCCGGGTGCAAAAGGTGATAAAGGTTCCAAGGGCGAAGTGGGTTTC
CCGGGTCTCGCGGGCAGCCCGGGCATTCCGGGGTCTAAGGGTGAGCAGGGTTTCAT
G (SEQ ID NO.6).
3. The successfully constructed expression plasmids were transformed into *E. coli* competent cells BL21(DE3). The specific process was as follows: (1) The *E. coli* competent cells BL21(DE3) were taken out from an ultra-low temperature refrigerator and placed on ice. 2 µL of plasmid to be transformed was measured and added into the *E. coli* competent cells BL21(DE3) when half-melted, and mixed slightly well for 2 to 3 times. (2) The mixture was placed on ice and subjected to ice bath for 30 min, and then to heat shock in a water bath at 42°C for 45 s to 90 s. After removal, the mixture was placed on ice and subjected to ice bath for 2 min. (3) The mixture was transferred to a biological safety cabin, and 700 µL of liquid LB medium was added, and cultured for 60 min under the conditions of 37°C and 220 rpm. (4) 200 µL of bacterial solution was measured and spread uniformly onto an LB plate containing kanamycin sulfate. (5) The plate was incubated in an incubator at 37°C for 15 to 17 h until colonies of uniform size grew.
4. From the transformed LB plate, 5 to 6 single colonies were picked out and put in a shake flask containing an antibiotic stock solution (100 mg/L of kanamycin sulfate), and cultured in a constant-temperature shaker at 220 rpm and 37°C for a period of time until it was foggy. The cultured shake flask was then cooled to 16°C to 30°C. After IPTG (at a final concentration of 0.5 mM) was added to induce expression for a period of time, the bacterial solution was sub-packed into centrifuge bottles, and centrifuged at 6000 rpm and 4°C for 12 min. The bacteria were collected, and the weight of the bacteria was recorded. Samples were taken for the electrophoresis test (the sample name was recorded as "bacterial solution").
5. The collected bacteria were re-suspended with an equilibrium working solution (200 mM sodium chloride, 25 mM Tris, 20 mM imidazole). The bacterial solution was cooled to ≤15°C, and subjected to homogenization twice or ultrasonic cell disruption. Upon completion, the bacterial solution was collected and subjected to the electrophoresis test (the sample names were recorded as "homogenization I", "homogenization II" or "ultrasound", respectively). The bacterial solution obtained after cell disruption was sub-packed into centrifuge bottles, and centrifuged at 17000 rpm and 4°C for 30 min. The supernatant was collected. The supernatant and precipitate were collected for the electrophoresis test (the sample names were recorded as "supernatant" or "precipitate", respectively).
6. The recombinant type IV humanized collagens were purified and enzyme digested. The specific process was as follows:
   (1) Capture: a. Equilibration of Column: The column was equilibrated with an equilibrium solution (200 mM sodium chloride, 25 mM Tris, 20 mM imidazole) and the flow rate was 10 mL/min. b. Sample Loading: The centrifuged supernatant was added to the column until the liquid was exhausted and the flow rate was 5 mL/min. The flow-through solution was measured for the electrophoresis test (the sample name was recorded as "flow-through"). c. Cleaning of Impurity Proteins: 100 mL of washing buffer (200 mM sodium chloride, 25 mM Tris, 20 mM imidazole) was added until the liquid was exhausted at a flow rate of 10 mL/min, and the flow-through solution resulting from impurities cleaning was measured for the electrophoresis test (the sample name was recorded as "impurity cleaning"). d. Collection of Target Proteins: 20 mL of eluent (200 mM sodium chloride, 25 mM Tris, 250 mM imidazole) was added at a flow rate of 10 mL/min, and the flow-through solution was collected. The protein concentration was tested by the ultraviolet-visible spectrophotometry and calculated based on the following equation: (C (mg/mL) = A280 × dilution multiple × extinction coefficient), and the protein was subjected to the electrophoresis test (the sample name was recorded as "elution"). e. The column was washed with 1M imidazole working solution and the flow rate was 10 mL/min. The flow-through solution was measured for the electrophoresis test (the sample name was recorded as "1M washing").
   (2) Enzyme digestion: TEV enzyme was added at a ratio of 20:1 between the total mass of proteins and the total mass of the TEV enzyme, and the samples were enzyme digested at 16°C for 2 h. Samples before and after the enzyme digestion were taken for the electrophoresis test (the sample names were recorded as "before digestion" and "after digestion", respectively). The digested protein solution was put into a dialysis bag, dialyzed at 4°C for 2 h, then transferred to a new dialysate (20 mM sodium chloride, 20 mM Tris), and dialyzed overnight at 4°C. The protein solution was sampled for the electrophoresis test (the sample name was recorded as "after diafiltration").
   (3) Polishing: a. Equilibration of Column: The column was equilibrated with liquid A (20 mM Tris, 20 mM sodium chloride), and the flow rate was 10 mL/min. b. Sample Loading: The flow rate was 5 mL/min. The sample was loaded and the flow-through solution was collected for the electrophoresis test (the sample name was recorded as "FL"). c. Gradient Elution: The gradients were set as 0% to 15% aqueous solution of liquid B for 2 min and then maintained at 3 CV, 15% to 30% aqueous solution of liquid B for 2 min and then maintained at 3 CV, 30% to 50% aqueous solution of liquid B for 2 min and then maintained at 3 CV, and 50% to 100% aqueous solution of liquid B for 2 min and then maintained at 3 CV were set respectively, in which liquid B contained 1M sodium chloride and 20 mM Tris, and the above percentages were volume fraction. After the peak occurred, the eluent was collected for the electrophoresis test (the sample name was recorded as "elution B"). d. Cleaning of Column. e. The target protein content was tested and the protein yield was calculated. The protein was preserved at 4°C.
   (4) Water Exchange: The collected protein solution was added to a 10 kDa ultrafiltration concentration tube, centrifuged at 3500 rpm for 15 min, and concentrated. The solution was changed. The operations were repeated three times. The electrophoresis test was conducted (the sample name was recorded as "water exchange").
7. Test Results: The electrophoresis test results involved in the preparation process of each of the recombinant proteins were shown in FIGS. 1 to 3. FIG. 4 showed a comparison of the stability of expression between the recombinant type IV humanized collagens C4P7C6 and C007.

FIG. 1 and FIG. 4 were the electrophoresis test results of the recombinant type IV humanized collagen C007, showing better purification and enzyme digestion effects.

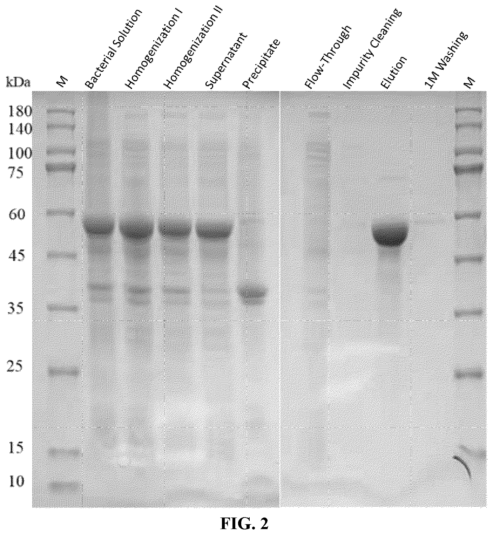

FIG. 2 and FIG. 4 were the electrophoresis test results of the recombinant type IV humanized collagen C4P7C6, showing better purification and enzyme digestion effects.

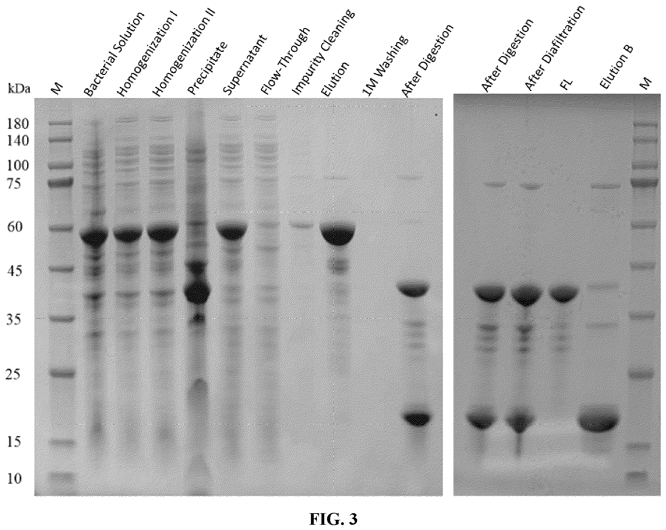

FIG. 3 was the electrophoresis test results of the recombinant type IV humanized collagen C4P7ES, showing better purification and enzyme digestion effects.

### Example 2: Bioactivity Assay of Recombinant Type IV Humanized Collagens

The test method for the activity of collagen could be found in the following literature: Juming Yao, Satoshi Yanagisawa, Tetsuo Asakura, Design, Expression and Characterization of Collagen-Like Proteins Based on the Cell Adhesive and Crosslinking Sequences Derived from Native Collagens, J Biochem. 136, 643-649(2004). The specific implementation method was as follows:
(1) The concentrations of the protein samples to be tested were detected by the ultraviolet absorption method, including commercial human collagen (Sigma, C7774) (as a positive control), and the recombinant type IV humanized collagens C4P7C6, C4P7E5, and C007 with better purification and enzyme digestion effects provided by the present disclosure.
   Specifically, the ultraviolet absorbance of a sample was determined at 215 nm and 225 nm respectively, and the protein concentration was calculated based on the empirical formula: C (µg/mL) = 144 × (A215 - A225). Of note, the test was conducted when A215 < 1.5. The principle of this method was as follows: determination of the characteristic absorption of peptide bonds under far-ultraviolet light was not subject to the chromophore content, experienced few interfering substances, was easy to operate, and suitable for detection of human collagen and its analogs that did not develop color with Coomassie Brilliant Blue. (Reference Literature: Walker JM. The Protein Protocols Handbook, second edition. Humana Press. 43-45.) After the protein concentrations were tested, the concentrations of all the proteins to be tested were adjusted with PBS to 0.5 mg/mL.
(2) 100 µL of each of the protein solutions and a PBS solution as blank control were added into a 96-well plate and left to stand still at room temperature for 60 min.
(3) To each well, 10⁵ well-cultured 3T3 cells were added and incubated at 37°C for 60 min.
(4) Each well was washed with PBS four times.
(5) The absorbance at OD₄₉₂ₙₘ was detected using an LDH Detection Kit (Roche, 04744926001). Based on the value of the blank control, the adherence rate of the cells could be calculated. The calculation formula was as follows: cell adherence rate = (test well-blank well)/(positive well-blank well) × 100%. The cell adherence rate could reflect the activity of collagen. The higher the activity of the protein, the more it could provide a high-quality external environment for cells in a short time and helped the cells adhere to the wall.

The results were as shown in FIG. 5. As could be appreciated from the comparison, the recombinant type IV humanized collagens of the present disclosure had a more excellent biological adherence activity as compared to the commercial human collagen, *i.e.:* the recombinant type IV humanized collagen C4P7C6 > C007 > C4P7E5 > human collagen.

### Example 3: Mass Spectrometry Detection of Recombinant Type IV Humanized Collagens

### Experimental Method:

**Table 1 Parameters for Mass Spectrometry Detector**

| | | | |
|---|---|---|---|
| **Instrument Name** | Matrix-Assisted Laser Desorption/Ionzation Time of Flight Mass Spectrometer MALDI-TOF/TOF Ultraflextreme^{™}, Brucker, Germany | | |
| **Matrix** | CHCA | **Laser energy** | 125 |
| **Data retrieval software** | Mascot | **Retrieval species** | ALL entries |
| **Retrieval database** | NCBIprot | | |

After reduced by DTT and alkylated with iodoacetamide, the protein sample was enzyme digested overnight with trypsin. The peptide fragments obtained after enzyme digestion were then desalted through C18ZipTip, mixed with the matrix α-cyano-4-hydroxycinnamic acid (CHCA), and spotted on a plate. Finally, the protein sample was assayed using a Matrix-Assisted Laser Desorption/Ionzation Time of Flight Mass Spectrometer MALDI-TOF/TOF UlraflextremeTM, Brucker, Germany (the technique of peptide fingerprint spectrum could be found in Protein J. 2016; 35:212-7).

Data retrieval was conducted on the MS/MS Ion Search page on the local Masco website. Protein identification results were obtained from the primary mass spectrum of the peptide fragments produced after enzyme digestion. Detection parameters: trypsin was used for enzyme digestion, and two missed cleavage sites were set. Alkylation of cysteine was set as a fixed modification. Oxidation of methionine was set as a variable modification. The database used for identification was NCBprot.

**Table 2 Mass-Spectrometry Detected Molecular Weight and Corresponding Polypeptides of C007**

| Observed Values | Mr (Predictive Values) | Peptides |
|---|---|---|
| 1343.6769 | 1342.6696 | GSVGGMGLPGTPGEK (SEQ ID NO.8) |
| 1629.8680 | 1628.8607 | GVPGIPGPQGSPGLPGDK (SEQ ID NO.9) |
| 1289.7395 | 1288.7323 | GQAGPPGIGIPGLR (SEQ ID NO.10) |
| 1603.9054 | 1602.8982 | GQAGPPGIGIPGLRGEK (SEQ ID NO. 11) |
| 2044.9894 | 2043.9821 | GEKGDQGIAGFPGSPGEKGEK (SEQ ID NO.12) |
| 1416.6853 | 1415.6780 | GDQGIAGFPGSPGEK (SEQ ID NO.13) |
| 1730.8411 | 1729.8338 | GDQGIAGFPGSPGEKGEK (SEQ ID NO.14) |
| 1367.7499 | 1366.7426 | GSIGIPGMPGSPGLK (SEQ ID NO.15) |
| 1383.7295 | 1382.7222 | GSIGIPGMPGSPGLK (SEQ ID NO.16) |
| 1642.8162 | 1641.8089 | GSPGSVGYPGSPGLPGEK (SEQ ID NO.17) |
| 1942.9584 | 1941.9511 | GSPGSVGYPGSPGLPGEKGDK (SEQ ID NO.18) |
| 1122.6582 | 1121.6509 | GLPGLDGIPGVK (SEQ ID NO.19) |
| 2695.4247 | 2694.4175 | GLPGLDGIPGVKGEAGLPGTPGPTGPAGQK (SEQ ID NO.20) |
| 1591.8157 | 1590.8084 | GEAGLPGTPGPTGPAGQK (SEQ ID NO.21) |
| 2121.0362 | 2120.0289 | GEPGSDGIPGSAGEKGEPGLPGR (SEQ ID NO.22) |
| 782.4179 | 781.4106 | GEPGLPGR (SEQ ID NO.23) |
| 877.4585 | 876.4512 | GFPGFPGAK (SEQ ID NO.24) |
| 1898.9974 | 1897.9901 | KGSKGEVGFPGLAGSPGIPGSK (SEQ ID NO.25) |
| 1626.8560 | 1625.8487 | GEVGFPGLAGSPGIPGSK (SEQ ID NO.26) |
| 2487.1361 | 2486.1288 | GEQGFMGPPGPQGQPGLPGSPGHATE (SEQ ID NO.27) |
| 2503.1228 | 2502.1155 | GEQGFMGPPGPQGQPGLPGSPGHATE (SEQ ID NO.28) |

The coverage rate of the polypeptide fragments was detected as 75.08%. The detection results were very reliable.

**Table 3 Mass-Spectrometry Detected Molecular Weight and Corresponding Polypeptides of C4P7E5**

| Observed Values | Mr (Predictive Values) | Peptides |
|---|---|---|
| 1333.7349 | 1333.6990 | GLPGTPGPTGPAGQK (SEQ ID NO.29) |
| 2672.4043 | 2672.3042 | GLPGTPGPTGPAGQKGEPGSDGIPGSAGEK (SEQ ID NO.30) |
| 3435.8640 | 3435.7019 | GLPGTPGPTGPAGQKGEPGSDGIPGSAGEKGEPGLPGR (SEQ ID NO.31) |
| 3590.9687 | 3590.7828 | GEVGFPGLAGSPGIPGSKGEQGFMGLPGTPGPTGPAGQK (SEQ ID NO.32) |
| 1983.0129 | 1982.9520 | GEQGFMGLPGTPGPTGPAGQK (SEQ ID NO.33) |
| 1999.0159 | 1998.9470 | GEQGFMGLPGTPGPTGPAGQK (SEQ ID NO.34) |
| 3321.7010 | 3321.5572 | GEQGFMGLPGTPGPTGPAGQKGEPGSDGIPGSAGEK (SEQ ID NO.35) |
| 2120.0861 | 2120.0134 | GEPGSDGIPGSAGEKGEPGLPGR (SEQ ID NO.36) |
| 781.4123 | 781.4082 | GEPGLPGR (SEQ ID NO.37) |
| 1639.8915 | 1639.8471 | GEPGLPGRGFPGFPGAK (SEQ ID NO.38) |
| 876.4635 | 876.4494 | GFPGFPGAK (SEQ ID NO.39) |
| 1176.6202 | 1176.5928 | GFPGFPGAKGDK (SEQ ID NO.40) |
| 2198.2251 | 2198.1332 | GDKGSKGEVGFPGLAGSPGIPGSK (SEQ ID NO.41) |
| 1898.0242 | 1897.9898 | GSKGEVGFPGLAGSPGIPGSK (SEQ ID NO.42) |
| 1625.8878 | 1625.8413 | GEVGFPGLAGSPGIPGSK (SEQ ID NO.43) |
| 2275.1863 | 2275.0943 | GEVGFPGLAGSPGIPGSKGEQGFM (SEQ ID NO.44) |

The coverage rate of the polypeptide fragments was detected as 100%. The detection results were very reliable.

**Table 4 Mass-Spectrometry Detected Molecular Weight and Corresponding Polypeptides of C4P7C6**

| Observed Values | Mr (Predictive Values) | Peptides |
|---|---|---|
| 876.4522 | 876.4494 | GFPGFPGAK (SEQ ID NO.45) |
| 3114.4779 | 3114.4771 | GEQGFMGPPGPQGQPGLPGSPGHAGFPGFPGAK (SEQ ID NO.46) |
| 3130.5134 | 3130.4720 | GEQGFMGPPGPQGQPGLPGSPGHAGFPGFPGAK (SEQ ID NO.47) |
| 2198.1324 | 2198.1332 | GDKGSKGEVGFPGLAGSPGIPGSK (SEQ ID NO.48) |
| 1897.9946 | 1897.9898 | GSKGEVGFPGLAGSPGIPGSK (SEQ ID NO.49) |
| 1625.8496 | 1625.8413 | GEVGFPGLAGSPGIPGSK (SEQ ID NO.50) |
| 2256.0358 | 2256.0382 | GEQGFMGPPGPQGQPGLPGSPGHA (SEQ ID NO.51) |
| 2272.0347 | 2272.0332 | GEQGFMGPPGPQGQPGLPGSPGHA (SEQ ID NO.52) |

The coverage rate of the polypeptide fragments was detected as 100%. The detection results were very reliable.

## Claims

1. A recombinant type IV humanized collagen, wherein an amino acid sequence of the recombinant type IV humanized collagen comprises n repeating sequence, n being an integer equal to or greater than 1; wherein the repeating sequence is a sequence of a functional domain of a native human type IV collagen;
preferably, n is 1, 2, 3, 4, 5, 6, 7 or 8, wherein when n is an integer equal to or greater than 2, each of the repeating sequences is directly linked;
more preferably, the amino acid sequence of the recombinant type IV humanized collagen comprises any one of the following (i) to (iii):
(i) an amino acid sequence set forth in SEQ ID NO.1, SEQ ID NO.2, or SEQ ID NO.3;
(ii) an amino acid sequence obtained by adding, substituting, deleting or modifying one or more amino acid residues in the amino acid sequence set forth in SEQ ID NO.1, SEQ ID NO.2, or SEQ ID NO.3 and retaining a cell adherence effect of the native human type IV collagen; and
(iii) an amino acid sequence that is encoded by a nucleotide sequence hybridized under a stringency condition with a polynucleotide sequence encoding the sequence set forth in SEQ ID NO.1, SEQ ID NO.2, or SEQ ID NO.3, and retains the cell adherence effect of the native human type IV collagen, the stringency condition being a medium stringency condition, a medium-high stringency condition, a high stringency condition or a very high stringency condition.

2. The recombinant type IV humanized collagen according to claim 1, wherein the amino acid sequence of the recombinant type IV humanized collagen further comprises an amino acid sequence excisable by a TEV protease, and the amino acid sequence excisable by the TEV protease is set forth in SEQ ID NO.7;
preferably, the amino acid sequence excisable by the TEV protease is directly linked to the repeating sequence;
preferably, the amino acid sequence excisable by the TEV protease is at N-terminus of the recombinant type IV humanized collagen.

3. A polynucleotide encoding the recombinant type IV humanized collagen according to claim 2,
preferably, a sequence of the polynucleotide comprising a sequence set forth in SEQ ID NO.4, SEQ ID NO.5, or SEQ ID NO.6.

4. A recombinant expression vector, wherein the recombinant expression vector comprises the polynucleotide according to claim 3,
preferably, the recombinant expression vector comprises a pET series vector, a shuttle vector, a phage or a viral vector;
more preferably, the recombinant expression vector is pET-28a-Trx-His.

5. A recombinant host cell, wherein the recombinant host cell comprises the recombinant expression vector according to claim 4,
preferably, the recombinant host cell is a prokaryotic cell, yeast or a eukaryotic cell;
more preferably, the recombinant host cell is *E. coli* BL21(DE3).

6. A preparation method for the recombinant type IV humanized collagen according to claim 1 or 2, comprising the following steps:
S1: subjecting the recombinant host cell according to claim 5 to fermentation culture, and inducing expression of a protein;
S2: collecting the protein; and
S3: purifying the protein; preferably, comprising purifying the protein by a Ni affinity chromatography column and/or an ion exchange chromatography column, and optionally subjecting the protein to enzyme digestion, preferably subjecting the protein to enzyme digestion by a TEV protease, more preferably, a mass ratio of the protein to the TEV protease being 15:1 to 25:1.

7. The preparation method according to claim 6, wherein in the step S1, the fermentation culture is carried out in a shaker at a rotational speed of 200 to 240 rpm and at a temperature of 35°C to 38°C.

8. The preparation method according to claim 6 or 7, wherein in the step S1, a specific procedure of inducing the expression of the protein is as follows: cooling to 16°C to 30°C and adding IPTG; preferably, the IPTG has a working concentration of 0.3 to 0.7 mM.

9. The preparation method according to any one of claims 6 to 8, wherein in the step S2, a specific procedure of collecting the protein is as follows: centrifuging a mixture obtained in the step S1 at 5000 to 7000 rpm for 10 to 15 min at 4°C, and re-suspending a precipitate with an aqueous solution containing 20 to 30 mM Tris, 150 to 250 mM sodium chloride, and 15 to 25 mM imidazole, and centrifuging at 15000 to 18000 rpm for 20 to 40 min at 4°C after high-pressure homogenization or ultrasonic disruption of the cells.

10. Use of the recombinant type IV humanized collagen according to claim 1 or 2 in a preparation of a product including a medical device, a cosmetic, a healthcare product, or a medicament.
